(19) Europäisches Patentamt
European Patent Office
Office européen des brevets

(11) Veröffentlichungsnummer: **0 124 562**
**B1**

# (12) EUROPÄISCHE PATENTSCHRIFT

(45) Veröffentlichungstag der Patentschrift: **08.06.88**

(51) Int. Cl.⁴: **C 07 H 21/00,** C 07 H 19/00

(21) Anmeldenummer: **83903508.6**

(22) Anmeldetag: **27.10.83**

(86) Internationale Anmeldenummer: **PCT/EP 83/00281**

(87) Internationale Veröffentlichungsnummer: **WO 84/01779 (10.05.84 Gazette 84/12)**

## (54) VERFAHREN ZUR HERSTELLUNG VON OLIGONUCLEOTIDEN.

(30) Priorität: **28.10.82 DE 3239887**

(43) Veröffentlichungstag der Anmeldung: **14.11.84 Patentblatt 84/46**

(45) Bekanntmachung des Hinweises auf die Patenterteilung: **08.06.88 Patentblatt 88/23**

(84) Benannte Vertragsstaaten: **AT BE CH DE FR GB LI LU NL SE**

(56) Entgegenhaltungen:
**EP-A-0 040 099**
**EP-A-0 061 746**

**Jakubke/Jeschkeit, Aminosäuren, Peptide, Proteine, Verlag Chemie (Weinheim 1982), Seiten 222-223**

(73) Patentinhaber: **BIOSYNTECH Biochemische Synthesetechnik GmbH, Stresemannstrasse 268-280, D-2000 Hamburg 50 (DE)**

(72) Erfinder: **KÖSTER, Hubert, Hallerstrasse 74, D-2000 Hamburg 13 (DE)**

(74) Vertreter: **Henkel, Feiler, Hänzel & Partner, Möhlstrasse 37, D-8000 München 80 (DE)**

**EP 0 124 562 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von Oligonucleotiden durch aufeinanderfolgende Verknüpfung einzelner oder mehrerer Nucleotideinheiten in homogener Phase, wobei nach jedem Verknüpfungsschritt eine chromatografische Aufarbeitung des Reaktionsgemisches mittels Molekularsieben erfolgt.

Infolge der Bedeutung synthetischer Oligonucleotide zur Gewinnung von genetischem Material sind verschiedene Verfahren zu deren Herstellung bekanntgeworden. Nach dem neuesten Stand der Technik werden diese Verfahren entweder nach der Phosphat-Triester- oder nach der Phosphit-Triester-Methode durchgeführt, wobei sich insbesondere die Verwendung polymerer Träger als heterogene Phase als vorteilhaft herausgestellt hat. Die Verwendung der polymeren Träger dient vor allem dazu, die Aufarbeitung der Reaktionsgemische nach den Kondensationsschritten zu vereinfachen, indem aufwendige chromatografische Trennverfahren durch Filtrationen ersetzt werden. Darüber hinaus ist auch eine Automatisierung der einzelnen Reaktionsschritte möglich. Diese Verfahren sind jedoch mit dem Nachteil behaftet, daß mit ihnen Oligonucleotide nur in relativ geringen Mengen zu gewinnen sind.

Aus H.-D. Jakubke und H. Jeschkeit "Aminosäuren, Peptide, Proteine", Verlag Chemie, Weinheim 1982, S. 222/223 ist es bekannt, bei der Peptidsynthese lösliche polymere Träger, z. B. Polystyrolträger, zu verwenden. Die Verwendung solcher hochmolekularer Träger bei der Oligonukleotidsynthese ist jedoch nicht erfolgversprechend, da die dabei erhaltenen Zwischenprodukte sich hinsichtlich ihres Molekulargewichts nur wenig von den jeweiligen Ausgangsmaterialien unterscheiden, was eine chromatographische Abtrennung der jeweils gebildeten Zwischenprodukte schwierig macht. Zudem neigen hochmolekulare Träger zur "Knäuelbildung", was Nachkupplungen erforderlich macht (aaO. S. 222, letzte Zeile). Darüber hinaus ist bei Einsatz hochmolekularer Träger mit wachsender Peptidkette auch eine Beeinflussung der Löslichkeitseigenschaften verbunden (aaO. der die Seiten 222 und 223 verbindene Satz). Im Gegesatz dazu ist der erfindungsgemäß verwendete Träger im Verhältnis zu den umzusetzenden Nukleotidbausteinen N sehr klein. Dies führt dazu, daß die im Verlauf der Synthese gewonnenen Zwischenprodukte immer oligonukleotidähnlicher werden und dadurch die Synthese in Bezug auf die chromatographische Aufarbeitung und die Löslichkeitseigenschaften erleichert wird.

Die Erfindung ist auf ein Verfahren der eingangs genannten Art gerichtet, mit dem Oligonucleotide definierter Sequenz in größeren Mengen erhalten werden können. Dabei sind nicht nur die in der Natur vorkommenden Oligodiester, sondern auch analoge Oligomere wie Oligotriester oder Oligophosphonate herstellbar.

Das Verfahren der Erfindung ist durch die folgenden Reaktionsschritte gekennzeichnet:

a) Umsetzung eines polyfunktionellen, in dem jeweiligen Reaktionsgemisch löslichen Trägermoleküls der allgemeinen Formel I

$$Sp(X)_n \qquad (I)$$

in der entweder Sp eine geradkettige oder verzweigte Polyalkylengruppe mit 2 bis 10 Kohlenstoffatomen und n eine ganze Zahl von 2-4 ist, oder

Sp eine ggf. verzweigte Alkylendioxygruppe mit 2-6 Kohlenstoffatomen und n = 2 ist, oder

Sp ein Pentaerythrolrest und n = 4 ist,

sowie X eine nucleotidchemisch verträgliche reaktive Gruppe ist, die mit den 3'- oder 5'-Hydroxygruppen eines Nucleosids, Nucleotids oder Oligonucleotids eine Silylether-, Tritylether- oder Esterbindung ausbilden kann, mit einer, bezogen auf $-(X)_n$, mindestens äquimolaren Menge eines ersten Nucleosids oder Nucleotids $N^1$,

b) Abtrennung und Reinigung sowie gegebenenfalls Phosphorylierung der in Stufe a erhaltenen Verbindung der allgemeinen Formel II

$$SP(X\text{-}N^1)_n \qquad (II)$$

c) Umsetzung der in Stufe b erhaltenen Verbindung II mit einer, bezogen auf $-(X\text{-}N^1)_n$ mindestens äquimolaren Menge eines zweiten Nucleosids oder Nucleotids $N^2$,

d) Abtrennung und Reinigung sowie gegebenenfalls Phosphorylierung der in Stufe c erhaltenen Verbindung der allgemeinen Formel III

$$Sp(X\text{-}N^1\text{-}N^2)_n \qquad (III)$$

sowie

e) gegebenenfalls weiteren aufeinanderfolgenden Verknüpfungs- und Reinigungs- sowie gegebenenfalls Phosphorylierungsschritten unter Bildung der Oligonucleotidverbindung der allgemeinen Formel IV

$Sp(X-N^1-N^2...N^m)_n$ (IV)

in der m eine ganze Zahl von 3-50 ist und $N^2$ $N^m$ für das zweite bis m-te Nucleosid bzw. Nucleotid steht, sowie

f) anschließende Freisetzung der Oligonucleotide der allgemeinen Formel V

$N^1-N^2...N^m$ (V)

in der m wie oben definiert ist.

Trägermoleküle mit geeigneten Resten X können z. B. in folgender Weise erhalten werden:

a) Mit Dicarbonsäureanhydriden, z. B. Bernstein- oder Adipinsärueanhydrid gemäß dem Reaktionsschema

$-Sp-OH + (OC-R-CO)_2O \rightarrow$

$-Sp-O-CO-R-COOH;$

b) mit Tritylderivaten gemäß dem Reaktionsschema

$-Sp-OH + HOOC-C_6H_4(C_6H_5)_2COH \rightarrow$

$-Sp-O-CO-C_6H_4(C_6H_5)_2C-OH \rightarrow -Sp-O-CO-C_6H_4(C_6H_5)_2C-Cl$

Diese Reaktion kann in Gegenwart eines Kondensationsmittels oder nach Überführung der Säurefunktion in eine Säurechloridfunktion erfolgen.

c) Mit Dichlorsiloxanen gemäß dem allgemeinen Schema

$-Sp-OH + Z(Me)_2Si-A-Si(Me)_2Z \rightarrow$

$-Sp-O-Si(Me)_2-A-Si(Me)_2Z,$

wobei A Alkylen, Arylen, -O- oder eine Kombination dieser Gruppen un Z Halogen ist.

d) mit Hydroxy-substituierten Tritylderivaten unter Bildung von Trityletherderivaten, z. B. der allgemeinen Formel

$-Sp-O-C_6H_4(C_6H_5)_2C-OH$ ($\rightarrow$ $-Sp-O-C_6H_4(C_6H_5)_2C-Cl$)

Bei der Verwendung der entsprechenden Thio- oder Amino-Derivate entstehen die entsprechenden S- bzw. N-Verbindungen.

Durch die Auswahl der Gruppe Sp ist gewährleistet, daß insbesondere das Trägermolekül $(Sp(X)_n$ und seine im Verlauf der Oligonucleotidsynthese der Erfindung erhaltenen Derivate in dem jeweils verwendeten Reaktionsmedium löslich und chromatografierbar sind.

Ein Beispiel für eine Alkylendioxyverbindungen Sp mit 1,4-Butandiol.

Die Gruppen $N^1$, $N^2...N^m$ bedeuten jeweils übliche Nucleotide, Nucleoside oder Oligonucleotide, die gegebenenfalls in geschützer Form eingesetzt bzw. im Verlauf der Synthese erhalten werden; typische Beispiele für derartige Schutzgruppen sind aus Tetrahedron 1981, Seite 363-369, Liebigs Ann. Chem. 1978, 839-853, sowie Nucleic Acids Research, Symposium Series No. 7, 1980, 39-59, ersichtlich.

Das Verfahren der Erfindung wird im folgenden näher erläutert, wobei auf die Reaktionsschemen Bezug genommen wird.

Kern des Verfahrens der Erfindung ist eine Synthesestrategie, die es ermöglicht, vorzugsweise das gewünschte Kondensationsprodukt von Ausgangs- und Nebenprodukten abtrennen zu können. Dies geschieht durch die Verwendung eines Trägermoleküls Sp als "purificaton handle", das über 2-4 funktionelle Gruppen verfügt, an denen im Verlauf des Verfahrens mehrere Oligonucleotidsynthesen gleichzeitig verlaufen. In den Reaktionsschemen bedeuten:

X = funktionelle Gruppe der angegebenen Definition
Sp = Trägermolekül bzw. Molekülteil der angegebenen Definition
$N^1$, $N^2$... = Nucleotidbausteine
$R^1$ = H,$OCH_3$
$R^2$ = z. b. 4-Nitrophenyl, Pentalchlorphenyl,
$R^3$ = H,OH oder geschütztes OH
$R^4$ = eine permanente Phosphatschutzgruppe, z. B. 2-Chlorophenyl-,
$R^5$ = eine temporäre Phosphatschutzgruppe, z. b. 2-Cyanethyl, 2,2,2-Trichlorethyl,
$R^6$ = Trityl, 4-Monomethoxytrityl, 4,4-Dimethoxytrityl,
$R^7$ = Alkyl (mit 1-20) Kohlenstoffatomen), Aryl, Aralkyl, Cycloalkyl, weiterhin 2-Cyanethyl, 2,2,2-Trichlorethyl,
$R^8$ = Acyl, insbesondere Acetyl, Laveolinyl-, β-Benzoylpropionyl, Aroyl, insbesondere Benzoyl, weiterhin o-Nitrobenzyl, Tetrahydropyranyl, Met oxytetrahydropyranyl, t-Butyldiphenylsilyl und dergleichen

W = Halogen, insbesondere Chlor oder Brom, Cyan,

Z = Halogen, insbesondere Chlor oder Brom, Cyan $N(R^7)_2$, insbesondere Dimethylamino; Triazolyl, Tetrazolyl, 3-Nitro-1,2,4-Triazolyl,

B = Nucleobase oder Analoga

Das Schema 1 zeigt allgemein ein Beispiel für ein bifunktionelles Trägermolekül der allgemeinen Formel I. Das unter Verwendung dieses Trägermoleküls durchgeführte Verfahren der Erfindung ergibt ein Kondensationsprodukt, das sich bei fortschreitender Kettenlänge in steigendem Maße von den niedermolekularen Nucleotidkomponenten und Nebenprodukten der Kondensationsreaktion im Molekulargewicht unterscheidet. Dies ermöglicht eine schonende Chromatografie unter Ausnutzung der unterschiedlichen Molekülgrößen.

Als Chromatografiematerialien kommen Molekularsiebe, z. B. auf Dextran- oder Polyamidbasis infrage. Da das gewünschte Produkt über das höchste Molekulargewicht verfügt, befindet es sich im Ausschlußvolumen und wird daher als erstes Produkt von der Chromatografiesäule eluieren, d. h. es wird zuerst und damit mit geringstem Zeitaufwand gewonnen. Da reversible Adsorptionserscheinungen zwischen Oligonucleotiden und Dextranen bzw. Polyamiden im Gegensatz zu Kieselgel nicht bekannt sind, ist auch der sonst übliche Verlust während der Chromatografie zu vernachlässigen.

Das Schema 2 gibt zwei prinzipielle Möglichkeiten wieder, ein bifunktionelles Trägermolekül mit den jeweils ersten Nucleotidbausteinen zu verbinden. Typ A zeigt zwei über das Trägermolekül miteinander verbrückte Tritylchloridgruppen, Typ B zeigt zwei über das Trägermolekül verbrückte Carboxylgruppen. Die Verwendung der Trägermoleküle vom Typ A oder Typ B hängt von der einzuschlagenden Oligonucleotidsynthesestrategie ab und wird im folgenden anhand der Raktionsschemen 3-8 näher erläutert.

Schema 3 zeigt die Synthese eines Oligonucleotids nach der Triesterkonzeption unter Verwendung des Trägermoleküls vom Typ A. Das makromolekulare Tritylchlorid wird mit einem Nucleosid-3'-phosphorsäuretriester umgesetzt, wobei $R^4$ in der Regel 2- oder 4-Chlorphenyl und $R^5$ 2-Cyanethyl oder 2,2,2-Trichlorethyl ist. Nach der Tritylierung erfolgt die Abspaltung der temporären Phosphatschutzgruppe $R^5$, und zwar im Falle von 2-Cyanethyl durch Umsetzung mit Triethylamin/Pyridin oder im Falle von 2,2,2-Trichlorethyl mit Acetylaceton/Zink, Triisopropylbenzolsulfonsäure oder Toluolsulfonsäure/Zink oder vorzugsweise mit Zink/Coreagens, wobei erfahrungsgemäß Anthranil-Säure, Pyridin-2-carbonsäure, Dithioerithrol oder Dithiothreitol eingesetzt werden können. Eine Kettenverlängerung erfolgt dann mit dem erhaltenen Diester als Phosphatkomponente und mit einem Nucleosid-3'-Triester als OH-Komponente unter Verwendung von Kondensationsmitteln wie z. B. Mesitylensulfonyl-3-nitro-1,2,4-triazol (MSNT). Das erhaltene voll geschützte Produkt wird über Molekularsiebchromatografie gereinigt; anschließend wird die Schutzgruppe $R^5$ abgespalten und ein erneuter Kettenverlängerungsschritt angeschlossen.

Die Verwendung des Trägermoleküls vom Typ B in der Triestersynthese wird in Schema 4 gezeigt. Das Nucleosid wird über die 3'-OH-Gruppe in diesem Falle esterartig an das Spacermolekül angekoppelt und dient als OH-Komponente. Diese wird mit einem 5'-tritylierten 3'-Nucleosid-diester unter Verwendung eines Kondensationsmittels, z. B. MSNT, zum voll geschützten Oligotriester umgesetzt. Es erfolgt eine Reinigung über Molekularsiebchromatografie und Abspaltung der 5'-Tritylgruppe entweder durch Verwendung einer Protonsäure oder einer Lewis-Säure wie $ZnBr_2$ oder Dialkylaluminiumchlorid.

Schema 5 zeigt die Verwendung des Trägermoleküls vom Typ A in der Phosphit-Triestersynthese. Die Kettenverlängerung wird eingeleitet, indem das an das Trägermolekül gebundene Nucleosid in der 3'-OH-Gruppe mit einem geschützten aktivierten Phosphit im ersten Schritt umgesetzt und im zweiten Schritt die verbliebene aktivierte Funktion mit der 5'-OH-Gruppe mit dem zweiten Nucleosid reagiert. Es folgt nun die Einführung von O, S oder Se, Umsetzung noch vorhandener 3'-OH-Gruppen durch Reaktion mit beispielsweise Essigsäureanhydrid oder Phenylisocyanat und anschließend, falls erforderlich, Abspaltung von $R^8$. Die Verwendung einer 3'-OH-Schutzgruppe ($R^8$) kann bei geeigneter Reaktionsführung entfallen, da die Regioselektivität die primäre OH-Gruppe stark begünstigt. Weitere Kettenverlängerungsschritte können sich anschließen.

Schema 6 gibt ein Beispiel für die Verwendung des Trägermoleküls vom Typ B in der Phosphit-Triestersynthese wieder. Hier wird das Nucleosid über die 3'-OH-Gruppe an dem Trägermolekül verankert, so daß die 5'-OH-Gruppe für eine Kettenverlängerung zur Verfügung steht. Die Kettenverlängerung wird eingeleitet durch Umsetzung mit einem 5'-tritylierten Nucleosidmonochloridit. Nach Koppelung der beiden Nucleoside erfolgt die Einführung von O, S oder Se, die Umsetzung noch vorhandener 5'-OH-Gruppen durch Reaktion mit beispielsweise Essigsäureanhydrid oder Phenylisocyanat und die Detritylierung mit Hilfe einer Protonsäure oder einer Lewis-Säure wie $ZnBr_2$ oder Dialkylaluminiumchlorid. Weitere Kettenverlängerungsschritte können sich anschließen.

Die Synthese von Oligophosphonaten unter Verwendung eines Trägermoleküls vom Typ A wird in Schema 7, diejenige unter Verwendung eines Träger-moleküls vom Typ B in Schema 8 beispielhaft wiedergegeben.

Das Verfahren der Erfindung wird im folgenden anhand der Synthese der Oligonucleotide d(TTTATT) und d(TTTATTCCT) näher erläutert. Die bei diesen Beispielen angewendete Synthesestrategie ergibt sich aus dem Schema 9; aus diesem Schema ist auch die Zusammensetzung der Ausgangs- und Zwischenprodukte ersichtlich. Eine Übersicht über weitere Verfahrensmerkmale der Kondensationsreaktionen gemäß den Beispielen ergibt sich aus Tabelle 1.

In Schema 9 wird anstelle der Abkürzung Sp für das Trägermolekül die Abkürzung LPC ("liquid phase carrier") verwendet.

**Ausführungsbeispiele**

Synthese der Oligodesoxynucleotide d(TTTAT) und d(TTTATTCCT) an einem LPC-Träger

1. Darstellung des "liquid phase carrier" (LPC)
1.1 Korksäurebrücke, über eine Esterbindung an Tritylgruppen verankert
1.1.1 Tritylierung
1.2 Korksäurebrücke, über eine Esterbindung an Dimethoxytritylgruppen verankert
1.2.1 Tritylierung
2. Elongationszyklus
2.1 Abspaltung der 3'-terminalen Trichlorethyl-Gruppe
2.2 Kondensationsreaktion mit Mono- oder Dimer-OH-Komponente
2.3 Gelfiltration an Sephadex LH 20
3. Entschützungsreaktionen
3.1 Oximat
3.2 Konz. $NH_3$
3.3 80 % $CH_3COOH$
4. Anionenaustauschchromatografie an DEAE-Cellulose
5. Sequenzierung

1.1 Darstellung von Dicarbonsäureestern (2a) aus Suberylchlorid (1a)

10 mmol des Alkohols (1a) in 20 ml Pyridin werden mit etwa 0,4 mmol des Suberylchlorids vorsichtig unter Eiskühlung und Rühren versetzt. Dann wird der Ansatz über Nacht bei Raumtemperatur stehengelassen. Anschließend wird er in Eiswasser gegossen und mit gesättigter Hydrogencarbonatlösung (1 : 1) versetzt. Nach dreimaliger Extraktion mit 50 ml $CHCl_3$ wird die gesammelte $CHCl_3$-Phase mit $Na_2SO_4$ getrocknet, filtriert und eingeengt. Endreinigung auf Kieselgel 60 H. Die Elution erfolgte mit reinem $CHCl_3$. Nach Einengung der das gewünschte Produkt enthaltenden Fraktionen erfolgte Fällung in Petrolether. Ausbeute: 70 %.
Schmp. 127°C.

| | | | | |
|---|---|---|---|---|
| Elementaranalyse berechnet: | C | 79.97 | gefunden: | 79.04 |
| | H | 6.13 | | 6.22 |

PMR/$CD_3$=D/ 1.38/Mulptiplett, 4 H, 4,4'-H 5,5'-H/, 1.71/Multiplett, 4H, 3,3'-H 6,6'-H/, 2.49/t, 4 H, 2,2'-H 7,7'-H/, 6.86-7.26/Multiplett, 28 Trityl H/.
IR/KBr/: 1740 $cm^{-1}$: -0-C=0; 3550 $cm^{1}$: -C-OH.

Herstellung des Tritylchlorids 3a durch Kochen von 2a in Toluol/Acetylchlorid

Zur Suspension von 3.168 mmol (2a) in 66 ml wasserfreiem Toluol wurde 1 ml frisch destilliertes Acetylchlorid gegeben und 6 Std. unter Rückfluß gekocht, wobei man nach 1 h nochmals 1,5 ml Acetylchlorid zusetzte. Der Ansatz wird im Wasserstrahlvakuum eingeengt. Der Rückstand wird in 40 ml Essigester aufgenommen und unter Rückfluß gekocht sowie zur langsamen Kristallisation stehengelassen. Die Kristalle werden filtriert und mit Ethylether gewaschen. Ausbeute: 90 % Schmp. 145°C.

| | | | | |
|---|---|---|---|---|
| Elementaranalyse: berechnet: | C | 75.92 % | gefunden: | 76.33 % |
| | H | 5.54 % | | 5.80 % |
| Hydrolysierbares | Cl | 9.74 % | | 9.72 % |

1.1.1 Darstellung von voll geschützten "monomeren-LPC-Derivaten" (5a) - Tritylierung von Thymidin 3'-(2-chlorophenyl-2,2,2-trichloroethyl)-phosphat (4)

2.75 mmol $T_dp$(te) (4) werden in 5 ml absolutem Pyridin gelöst, eingeengt und nochmals in 3 ml abs. Pyridin gelöst. 1.16 mmol des Dichlorids (3a) werden in 4 ml abs. Pyridin gelöst und zur Lösung 4 gegeben. Dann wird der Ansatz 3,5 h im Ölbad auf 100°C erhitzt und dünnschichtchromatografisch im Laufmittel $CHCl_3$/MeOH (95 : 5) verfolgt. Die Reaktion wird durch Zugabe von EtOH abgebrochen, mehrmals mit Toluol/$CHCl_3$ (3 : 7) eingeengt und schonend im Wasserstrahl- und Hochvakuum bei 30°C zu einem Schaum oder Sirup eingeengt. Die Endreinigung erfolgt durch Chromatografie mit 50 g Kieselgel pro Gramm zu reinigenden Materials. Elution mit $CHCl_3$, enthaltend 0-5 % MeOH. Die so gereinigte Substanz wird nach Einengen in 5 ml Dioxan gelöst und gefriergetrocknet. Ausbeute: 44 %.

1.2 Darstellung von 3-Hydroxy-4,4-dimethoxytriphenyl-carbinol (1b)

A. Darstellung der Grignard-Verbindung

In einem 2-l-Dreihalskolben mit Tropftrichter, Rührer und Rückflußkühler mit Calciumchloridrohr werden 2 mol Magnesiumspäne mit 200 ml THF (absolut) übergossen und mit etwa einem Zwanzigstel von insgesamt 2

mol (12,5 ml) Arylhalogenid (4-Bromanisol) unter Rühren versetzt.

Das Anspringen der Reaktion bemerkt man an dem Auftreten einer leichten Trübung und Erwärmung des Ethers. Sollte die Reaktion nicht einsetzen, gibt man zum Reaktionsgemisch 0,5 ml Brom oder einige Tropfen Tetrachlorkohlenstoff und erwärmt leicht. Nach dem Anspringen wird das restliche Arylhalogenid gelöst (235 ml 4-Bromanisol in 500 ml THF) und unter weiterem Rühren so zugetropft, daß der Ether gelinde siedet. Wird die Reaktion zu heftig, so kühlt man den Kolben mit Wasser. Gegen Ende des Eintropfens wird auf einem Wasserbad zum geringen Sieden erhitzt, bis praktisch alles Magnesium gelöst ist (etwa 30 minuten).

B. Umsetzung der Grignard-Verbindung mit dem Carbonsäureester

In die Grignard-Reagens-Lösung aus 2.0 mol Halogenid (4-Bromanisol) tropft man unter Rühren 0,6 mol 3-Hydroxybenzoesäuremethylester in 200 ml absolutem THF zu. Nach beendeter Zugabe erhitzt man unter Rühren noch 2 h, kühlt ab, hydrolisiert durch Zugabe von 100-150 g zerstoßenem Eis und gibt anschliessend so viel halbkonzentrierte Salzsäure zu, daß sich der entstandene Niederschlag gerade löst. Danach wird die Reaktionsmischung 3 mal mit 300 ml Ethylether ausgeschüttelt. Die etherische Schicht wird abgetrennt und die wäßrige Phase noch zweimal mit Ether extrahiert. Die vereinigten Extrakte werden mit gesättigter Hydrogencarbonatlösung und wenig Wasser gewaschen. Nach dem Trocknen über Natriumsulfat destilliert man den Ether ab. Dünnschichtchromatografie (DC) im Laufmittel $CHCl_3$/MeOH (95 : 5) zeigte das gewünschte und ein schneller laufendes, an der 3-OH-Gruppe verestertes Produkt. Der Ansatz wurde daher in 200 ml Pyridin aufgenommen, mit 400 ml konzentriertem $NH_3$ versetzt und die Suspension unter Rühren über Nacht stehengelassen, eingeengt (Hochvakuum) und in Pyridin aufgenommen (370 ml). Diese Lösung wird für den nächsten Schritt benutzt.

Darstellung von Dicarbonsäureestern (2b) aus Suberylchlorid und (1b)

0,28 mol (1b), gelöst in 205 ml Pyridin, werden im Hochvakuum eingeengt, mit etwa 0,112 mol des Suberylchlorids in 105 ml Pyridin vorsichtig unter Eiskühlung und Rühren versetzt. Dann wird der Ansatz über Nacht bei Raumtemperatur stehengelassen, im Hochvakuum (Ölpumpe) eingeengt und in 200 ml $CHCl_3$ aufgenommen. Nach dreimaliger Extraktion mit 150 ml gesättigter $NaHCO_3/H_2O$ (1 : 1) wird die $CHCl_3$-Phase mit $Na_2SO_4$ getrocknet, filtriert und eingeengt. Endreinigung erfolgt durch Kieselgelchromatografie. Elution mit reinem $CHCl_3$. Die das gewünschte Produkt enthaltenden Fraktionen werden direkt weiterverarbeitet.

Darstellung des Tritylchlorids 3b durch Kochen von 2b in Toluol Acetylchlorid

Zur Suspension von 5 mmol (2b) in 40 ml wassefreiem Toluol werden 10 ml Acetylchlorid gegeben und 4,5 h unter Rückfluß gekocht, wobei man nach 1 h nochmals 5 ml Acetylchlorid zusetzt. Der Ansatz wird im Wasserstrahlvakuum eingeengt, in Essigester aufgenommen und bei 0° C aufbewahrt.

1.2.1 Darstellung von voll geschützten "monomeren-LPC Derivaten" (5b) - Tritylierung von 4 und 3b in Gegenwart von Molekularsiebpulver

5 mMol des zu tritylierenden $T_d$-Derivates (4) werden in 8,5 ml Pyridin gelöst, eingeengt und nochmals in 4,0 ml Pyridin gelöst. Danach werden 20 ml $CH_2Cl_2$ zugesetzt.

Nach Zugabe von 6 g frisch aktiviertem gepulverten Molekularsieb 0,4 mm (4 Å) 30 Minuten Rühren und Zugabe von 2,5 mmol (2b) in 3,5 ml Pyridin wird das Reaktionsgemisch über Nacht gerührt und dünnschichtchromatografisch im Laufmittel $CHCl_3$/MeOH (95 : 5) untersucht.

Die Reaktion wird durch Zugabe von 10 ml EtOH abgebrochen, vom Molekularsieb abfiltriert, mit $CHCl_3$ nachgewaschen, das Filtrat zweimal mit 50 ml $H_2O$ gewaschen, die $CHCl_3$-Phase mit $Na_2SO_4$ getrocknet, filtriert und schonend im Wasserstrahl- und Hochvakuum bei 30° C zum Schaum oder Sirup eingeengt.

Endreinigung erfolgt durch Kieselgelchromatografie mit 50 g Kieselgel pro Gramm zu reinigenden Materials. Elution mit $CHCl_3$ enthaltend 2 % MeOH. Die so gereinigte Substanz wird nach Einengen des Lösungsmittels in Dioxan gelöst und gefriergetrocknet.

Ausbeute: 71 %.

2. Elongationszyklus

2.1 Abspaltung der 3'-terminalen Trichloroethyl-Gruppe

Pro mMol Triester in 15-20 ml Pyridin werden nacheinander unter magnetischem Rühren Anthranilsäure (10 Equiv.) und aktiviertes Zinkpulver (10 Equiv.) zugegeben. Die Reaktion wird dünnschichtchromatografisch im Laufmittel $CHCl_3$/MeOH (9 : 1) und (6 : 4) verfolgt. Nach 30 Minuten erfolgt Zugabe einer Mischung von $H_2O$/Triethylamin/Pyridin (0,3/0,625/l). Nach weiteren 10 Minuten Rühren wird über eine Glasfritte direkt in einen Scheidetrichter mit 125 ml gesättigter $NaHCO_3$-Lösung vom Zink abfiltriert, mit 10 ml Pyridin nachgewaschen, viermal mit 50 ml Chloroform extrahiert, die organische Phase gesammelt und dreimal mit 50 ml gesättigter $NaHCO_3$-Lösung gewaschen. Nach Reextraktion der wäßrigen Phase mit 50 ml Chloroform wird die organische Phase mit $Na_2SO_4$ getrocknet, filtriert und eingeengt.

Bei der Verbindung (5b) erfolgt die Endreinigung auf RP 2-Säule mit dem Elutionsmittel $MeOH/H_2O$ (8 : 2). Bei den größeren Molekülen wird der gereinigte LPC-Oligo-nucleotid-3'-phosphorsäurediester direkt zur

Kondensation eingesetzt.

2.2 Kondensationsreaktion mit monomeren oder dimeren OH-Komponenten

OH-Komponenten (dreifacher Überschuß über LPC-Phosphatkomponente) und LPC-Phosphatkomponenten werden zwecks azeotroper Trocknung mit je 10 ml Pyridin pro mMol Phosphatkomponente zweimal zum Schaum und einmal bis zum ca. halben Volumen koevaporiert, mit 4,5-5 äquivalenten 1-(Mesitylen-2-sulfonyl)-3-nitro-1,2,4-triazol (MSNT) versetzt und unter DC-Kontrolle 1.5 h abhängig von der Kettenlänge, magnetisch gerührt. Zum Reaktionsabbruch wird der Ansatz mit 1,5 ml gesättigter $NaHCO_3$-Lösung versetzt und mit 50 ml $CHCl_3$ in einen Scheidetrichter mit 75 ml gesättigter $NaHCO_3$-Lösung überführt. Nach sechsmaliger Extraktion mit je 30 ml $CHCl_3$ wird die gesammelte $CHCl_3$-Phase mit $Na_2SO_4$ getrocknet, filtriet und eingeengt. Endreinigung auf Sephadex® LH 20.

2.3 Gelfiltration an Sephadex LH 20

Die Endreinigung der Reaktionsmischung nach der Kondensationsreaktion erfolgt an Sephadex LH 20 mit dem Laufmittel $CHCl_3$/ EtOH (7 : 3). Es wird eine Säule mit 63 x 3,6 cm (640 $cm^3$, Ausschlußvolumen 200 ml) benutzt. Durchflußgeschwindigkeit 120 ml/ Std., Fraktionen zu je 2 ml.

Die Bestandteile der Kondensationsreaktionsmischung werden in folgender Reihenfolge eluiert: Kondensationsprodukte (Fraktionen 1-20), unreagierte Phosphatkomponente (15-35), sulfonylierte OH-Komponente (30-50), unreagierte OH-Komponente (40-75).

Nur bei den längeren Ketten, wo relativ große LPC-Phosphatkomponenten zur Kondensationsreaktion eingesetzt wurden, kann gelegentlich ein Überlappen zwischen unreagierten LPC-Phosphatkomponenten und Kondensationsprodukten beobachtet werden. In diesen Fällen erfolgte Filtration über eine sehr dünne Schicht von Kieselgel, 60 H (Merck).

3. Entschützungsreaktionen

Hexamer TTTATT wird nach Sephadex LH 20-Chromatografie über Kieselgel filtriert.

Nonamer TTTATTCCT wird nach Sephadex LH 20-Chromatografie dünnschichtchromatografisch an RP-18 im Laufmittel Aceton/$H_2O$ (75 : 25) gereinigt.

3.1 Abspaltung der Phosphatschutzgruppen mit Oximat

Das gereinigte Material wird in 200 µl Dioxan gelöst und mit 0.2 mmol Pyridin-2-carboxaldoxim (24,4 mg), 0.2 mmol Tetramethyl guanidin (25 µl) sowie 200 µl Wasser versetzt. Nach 24 h erfolgt eine Zugabe von weiteren 0.2 mmol Tetramethylguanidin und weitere 26 h Reaktionszeit bei Raumtemperatur.

3.2 Abspaltung der N- und O-Schutzgruppen mit konz. $NH_3$

Anschließend wird das Gemisch mit 0.85 ml konz. wäßrigem $NH_3$ verstzt, die erhaltene homogene Lösung dicht verschlossen, 24 h auf 50°C gehalten und unter schonenden Bedingungen eingeengt.

3.3 Spaltung der Tritytetherbindung mit 80 % $CH_3COOH$

Das so erhaltene Material wird in 1 ml 80 % Essigsäure aufgenommen und 2,5 h auf Raumtemperatur gehalten. Danach erfolgt Einengen durch Gefriertrocknung, Lösung in 400 µl $H_2O$ und Extraktion mit $CHCl_3$. Die wäßrige Phase wird mit $NH_3$ neutralisiert. Die so erhaltene Mischung wird mit 10 ml 25 mM Tris/HCL Puffer (pH 7.5)/7 M Harnstoff verdünnt. Die Endreinigung erfolgt durch DEAE-Cellulose/Anionenaustauschchromatografie.

4. Anionenaustauschchromatografie an DEAE-Cellulose

Das entschützte Material wird aus verdünnter Lösung auf eine mit 25 mM Tris/HCl Puffer (pH 7.5)/7 M Harnstoff äquilibierte DEAE-Cellulosesäule (12 cm lang, 21 $cm^3$ Inhalt) aufgezogen. Danach erfolgt gründliches Waschen mit 50 mM NaCl im gleichen Puffer und Elution mit einem Ionenstärkegradienten von 50 mM bis 500 mM NaCl, ebenfalls im gleichen Puffer. Das Hexanucleotid wird bei einer NaCl-Konzentration von ca. 0.225 mM NaCl eluiert. Das Nonanucleotid wurde bei einer NaCl-Konzentration von ca. 0.285 mM NaCl eluiert.

5. Sequenzanalyse

Die dann durchgeführte Sequenzanalyse wurde nach der "mobility shift method" durchgeführt.
Literatur:

F. Sanger et al., PNAS 70, 1209 (1973).
E. Jayet al., NAR 1, 331-52 (1974).

H. Blöcker and H. Köster, Liebig's Ann. Chem. 1978, 982-90.

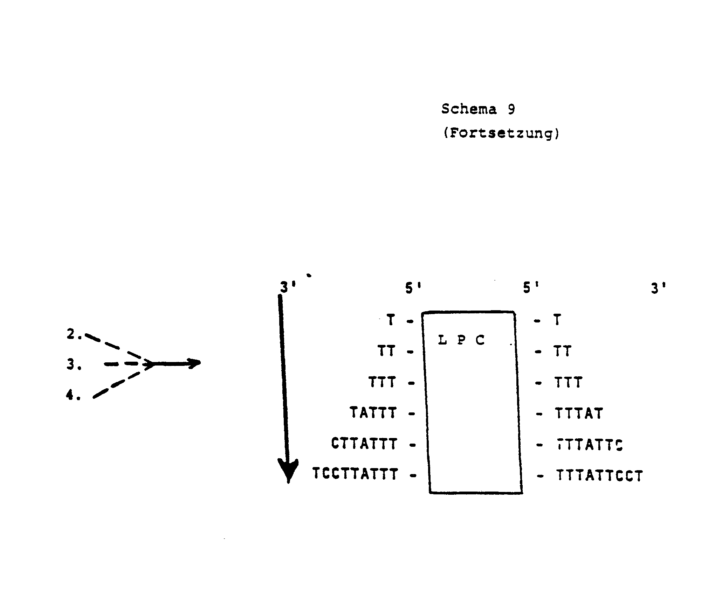

**Patentanspruch**

Verfahren zur Herstellung von Oligonucleotiden durch aufeinanderfolgende Verknüpfung einzelner oder mehrerer Nucleosid- und/ oder Nucleotideinheiten in homogener Phase, wobei nach jedem Verknüpfungsschritt eine chromatografische Aufarbeitung des Reaktionsgemisches mittels Molekularsieben erfolgt, gekennzeichnet durch die folgenden Reaktionsschritte:

a) Umsetzung eines bi- bis tetrafunktionellen, in dem jeweiligen Reaktionsgemisch löslichen Trägermoleküls der allgemeinen Formel I

$$Sp(X)_n \qquad (I)$$

in der entweder Sp eine geradkettige oder verzweigte Polyalkylengruppe mit 2 bis 10 Kohlenstoffatomen und n eine ganze Zahl von 2-4 ist, oder

Sp eine ggf. verzweigte Alkylendioxygruppe mit 2-6 Kohlenstoffatomen und n = 2 ist, oder

Sp ein Pentaerythrolrest und n = 4 ist,

sowie X eine nucleotidchemisch verträgliche reaktive Gruppe ist, die mit den 3'- oder 5'-Hydroxygruppen eines Nucleosids, Nucleotids oder Oligonucleotids eine Silylether-, Tritylether oder Esterbindung ausbilden kann, mit einer, bezogen auf $-(X)_n$, mindestens äquimolaren Menge eines ersten Nucleosids oder Nucleotids $N^1$,

b) Abtrennung und Reinigung sowie gegebenenfalls Phosphorylierung der in Stufe a erhaltenen Verbindung der allgemeinen Formel II

$$Sp(X\text{-}N^1)_n \qquad (II)$$

c) Umsetzung der in Stufe b erhaltenen Verbindung II mit einer, bezogen auf $-(X\text{-}N^1)_n$, mindestens äquimolaren Menge eines zweiten Nucleosids oder Nucleotids $N^2$,

d) Abtrennung und Reinigung sowie gegebenenfalls Phosphorylierung der in Stufe c erhaltenen Verbindung der allgemeinen Formel III

$$Sp(X\text{-}N^1\text{-}N^2)_n \qquad (III)$$

sowie

e) gegebenenfalls weiteren aufeinanderfolgenden Verknüpfungs- und Reinigungs- sowie gegebenenfalls Phosphorylierungsschritten unter Bildung der Oligonucleotidverbindung der allgemeinen Formel IV

$$Sp(X\text{-}N^1\text{-}N^2 \ldots N^m)_n, \qquad (IV)$$

in der $N^2 \ldots N^m$ für das zweite bis m-te Nucleosid bzw. Nucleotid steht und m eine ganze Zahl von 3-50 ist, sowie

f) anschließende Freisetzung der Oligonucleotide der allgemeinen Formel V

$$N^1\text{-}N^2 \ldots N^m \qquad (V)$$

in der m wie oben definiert ist.

**Claim**

Process for preparing oligonucleotides by successively linking together individual or several nucleoside and/or nucleotide units in a homogenous phase system and by which the reaction mixture is chromatographically purified by means of molecular sieves after each linking stage. The process is characterized by the following reaction stages:

a) reaction of a bi- to tetrafunctional carrier molecule with the general formula I

$$Sp(X)_n \qquad (I)$$

that is soluble in the corresponding reaction mixture, in which Sp is either a straight chain or branched polyalkylene group with 2 to 10 carbon atoms and in which n is an integer between 2 and 4, or

in which Sp, if appropriate, is a branched alkylenedioxy group with 2 to 6 carbon atoms and n = 2, or

in which Sp is a pentaerythrol residue and n = 4,

and X is a compatible reactive group in terms of nucleotide chemistry that can form a silyl ether, trityl ether or an ester bond with the 3' or 5' hydroxyl groups of a nucleoside, nucleotide or an oligonucleotide with an amount of the initial nucleoside or nucleotide $N^1$ that is at least equimolar to $-(X)_n$

b) separation and purification and, if appropriate, phosphorylation of the compound obtained in stage a with the general formula II

$$Sp(X\text{-}N^1)_n \tag{II}$$

c) reaction of compound II obtained from stage b with at least an equimolar amount, in terms of $-(X\text{-}N_1)_n$, of a second nucleoside or nucleotide $N^2$,

d) separation and purification and, if necessary, phosphorylation of the compound obtained in stage c with the general formula III

$$Sp(X\text{-}N^1\text{-}N^2)_n \tag{III}$$

e) and, if necessary, additional successive linking and purification and, if appropriate, phosphorylation stages to form the oligonucleotide compound with the general formula IV

$$Sp(X\text{-}N^1\text{-}N^2...N^m)_n, \tag{IV}$$

in which $N^2$... $N^m$ represents the second to the mth nucleoside or nucleotide respectively, and m is an integer between 3 and 50,

f) subsequent release of the oligonucleotide with the general formula V

$$N1\text{-}N^2...Nm \tag{V}$$

in which m is as defined above..

**Revendication**

Procédé de production d'oligonucléotides par greffage successif d'une seule ou de plusieurs unités nucléosides et/ou nucléotides en phase homogène, en réalisant après chaque étape de greffage un traitement chromatographique du mélange réactionnel au moyen de tamis moléculaires, caractérisé par les étapes réactionnelles suivantes:

a) Réaction d'une molécule porteuse soluble dans le mélange réactionnel considéré, bi- à tétrafonctionnelle, de formule générale I

$$Sp(X)_n \tag{I}$$

dans laquelle Sp est soit un groupe polyalkylène linéaire ou ramifié avec 2 à 10 atomes de carbone et n est un nombre entier de 2 à 4, soit

Sp est un groupe alkylène-dioxy ramifié le cas échéant avec 2 à 6 atomes de carbone et n est 2, soit

Sp est un reste pentaérythrol et n = 4 de même X est un groupe réactif compatible chimiquement avec les nucléotides, qui peut former avec les groupes hydroxy 3'- ou 5' d'un nucléoside, nucléotide ou oligonucléotide une liaison silyléther, trityléther ou ester, avec une quantité au moins équimolaire, rapportée à $-(X)_n$, d'un premier nucléoside ou nucléotide $N^1$,

b) Séparation et purification ainsi que phosphorylation le cas échéant du composé obtenu à l'étape a de formule générale II

$$Sp(X\text{-}N^1)_n \tag{II}$$

c) Réaction du composé II obtenu à l'étape b avec une quantité au moins équimolaire, rapportée à $-(XN^1)_n$, d'un deuxième nucléoside ou nucléotide $N^2$,

d) Séparation et purification ainsi que phosphorylation le cas échéant du composé obtenu à l'étape c de formule générale III

$$Sp(X\text{-}N^1\text{-}N^2)_n \tag{III}$$

ainsi que

e) le cas échéant d'autres étapes successives de greffage et de purification ainsi que de phosphorylation le cas échéant en formant le composé oligonucléotide de formule générale IV

$$Sp(X\text{-}N^1\text{-}N^2 \ldots N^m)_n, \qquad (IV)$$

dans laquelle $N^2 \ldots N^m$ représente du deuxième au m-ième nucléoside ou nucléotide et m est un nombre entier de 3 à 50, ainsi que

f) Libération ultérieure de l'oligonucléotide de formule générale V

$$N^1\text{-}N^2 \ldots N^m \qquad (V)$$

dans laquelle m est défini comme ci-dessus.

Tabelle 1: Kondensationsreaktionen

| Phosphat-Komponente | (mMol) | OH-Komponente | (mMol) | h | MSNT (mMol) | Kondensationsausbeute (%) Produkt |
|---|---|---|---|---|---|---|
| LPC-$(Tp^-)_2$ | 1,00 | Tp(te) | 3,00 | 1 | 4,50 | LPC-$(TpTp(te))_2$ (81) |
| LPC-$(TpTp^-)_2$ | 0,57 | Tp(te) | 1,72 | 1 | 2,60 | LPC-$(TpTpTp(te))_2$ (57,5) |
| LPC-$(TpTpTp^-)_2$ | 0,37 | $A^{bz}pTp(te)$ | 0,62 | 1 | 1,67 | LPC-$(TpTpTpA^{bz}pTp(te))_2$ (30) |
| LPC-$(TpTpTpA^{bz}Tp^-)_2$ | 0,04 | $TpC^{tl}p(te)$ | 0,10 | 2 | 0,20 | LPC-$(TpTpTpA^{bz}pTpTpC^{tl}p(te))_2$ (35) |
| LPC-$(TpTpTpA^{bz}pTpTpC^{tl}p^-)_2$ | 0,008 | $C^{tl}pT(Bb)$ | 0,02 | 5 | 0,04 | LPC-$(TpTpTpA^{bz}pTpTpC^{tl}pCpT(Bb))_2$ (-) |
| LPC-$(TpTpTpA^{bz}pTp^-)_2$ | 0,03 | T(Bb) | 0,105 | 4 | 0,15 | LPC-$(TpTpToA^{bz}pTpT(Bb))_2$ (56) |

te: 2,2,2-Trichloroethyl

bz: Benzoyl

tl: 2-Toluyl

Bb: 4-tert-butylbenzoyl

Schema 1

$X - Sp - X$

$\downarrow N^1$

$N^1 - X - Sp - X - N^1$

$\downarrow N^2$

$N^2 - N^1 - X - Sp - X - N^1 - N^2$

$\downarrow$ usw.

Schema 2

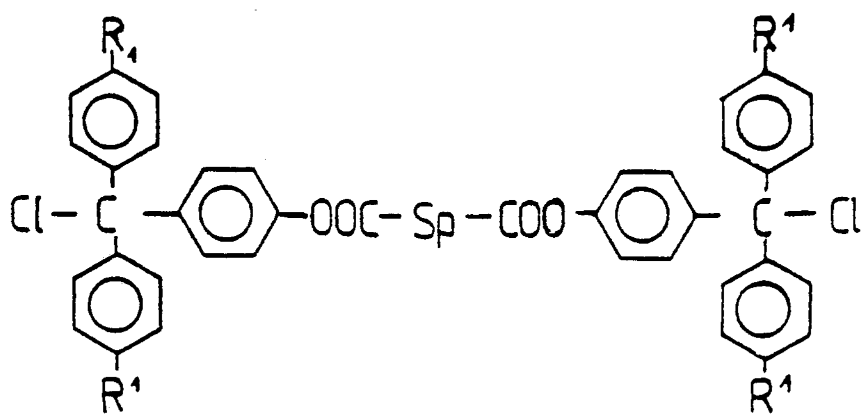

A

$X-OC-Sp-CO-X$

B

Schema 3

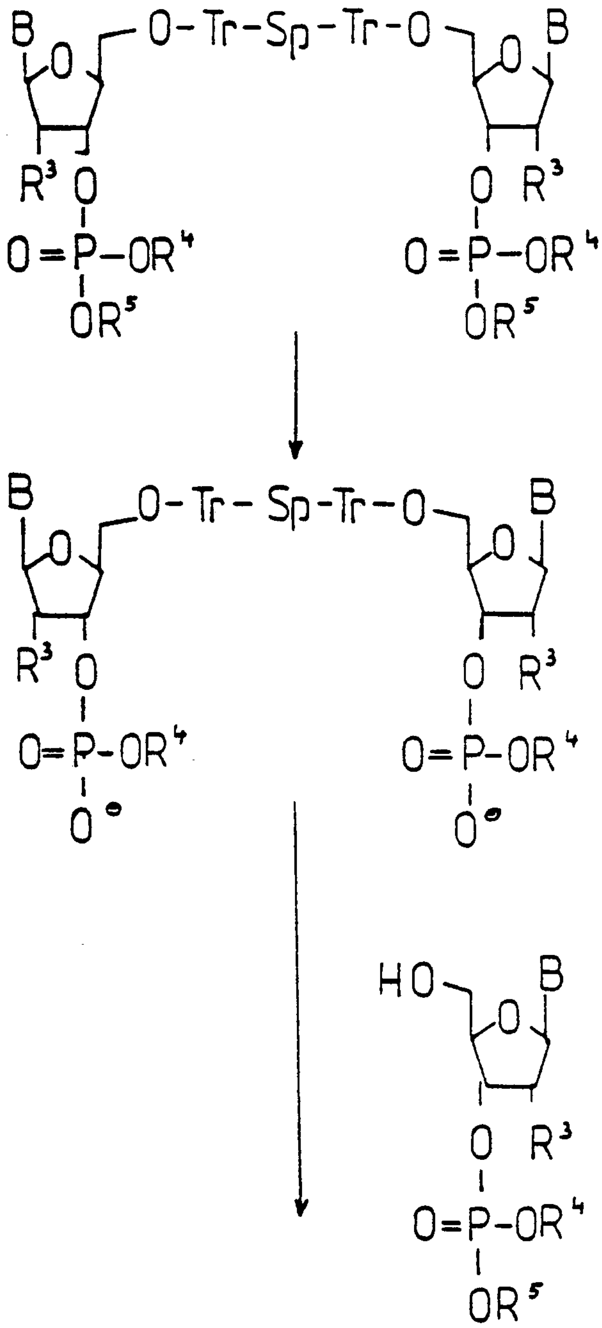

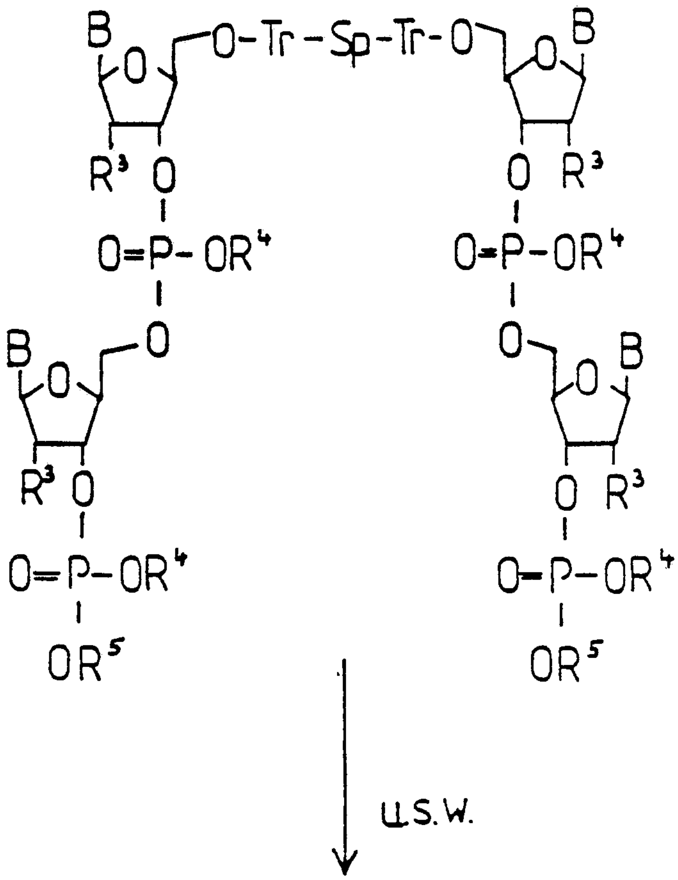

(Fortsetzung)

Schema 7

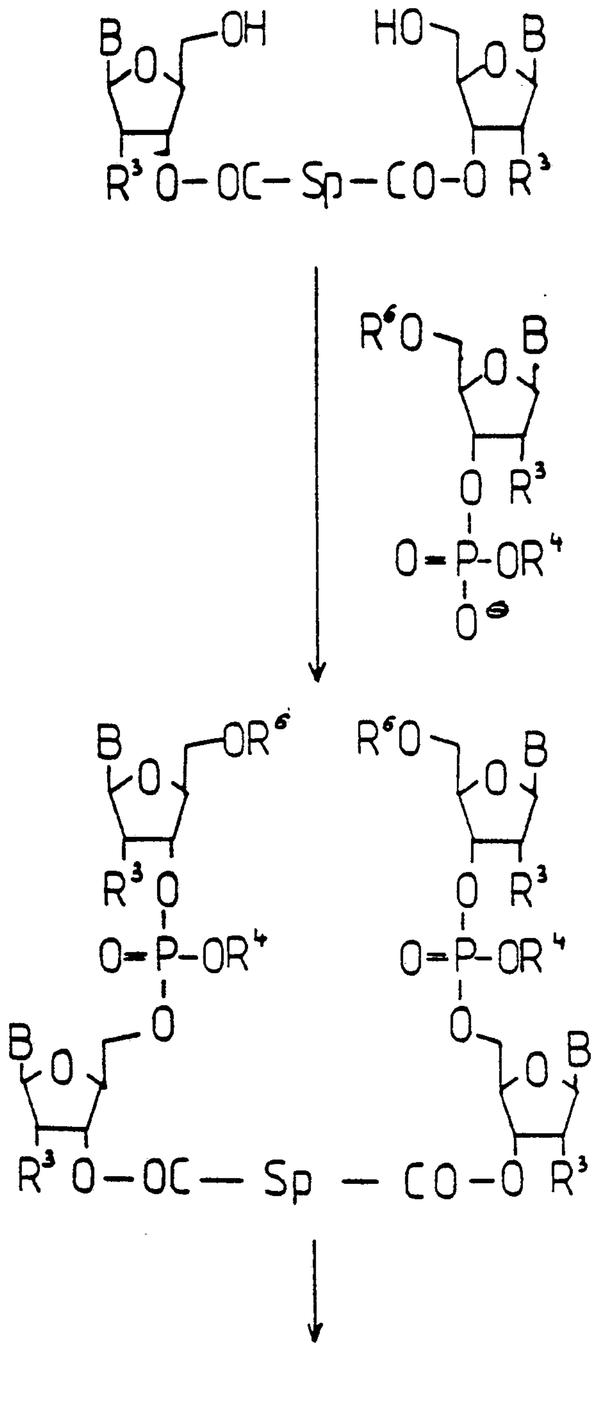

Schema 4
(Fortsetzung)

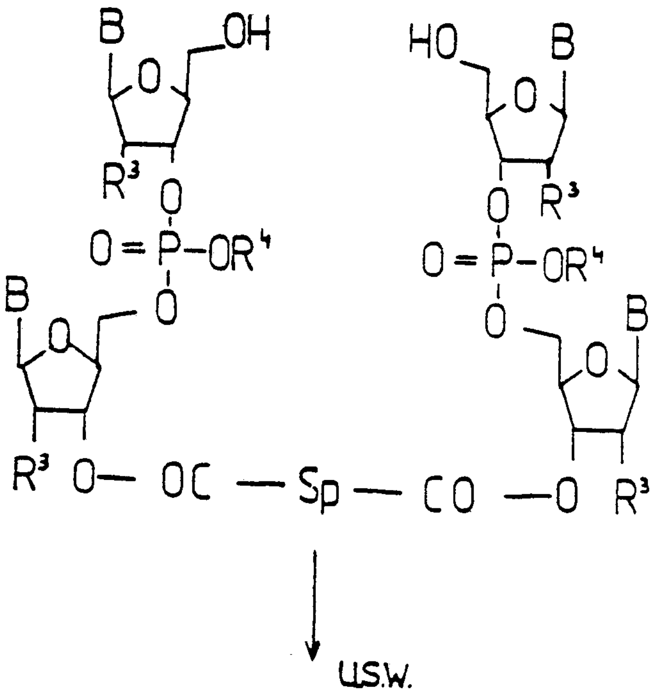

Schema 5

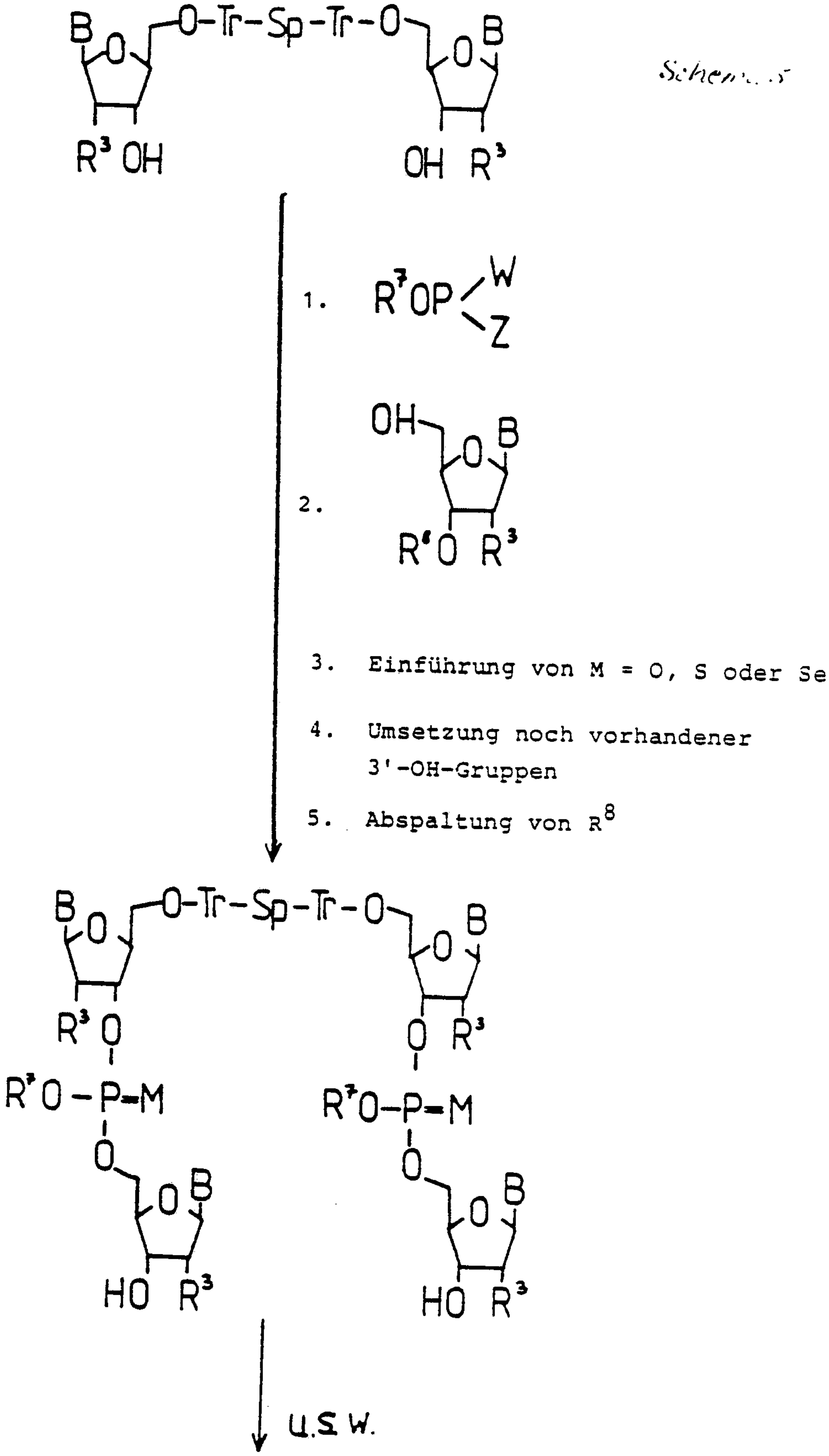

1.
2.
3. Einführung von M = O, S oder Se
4. Umsetzung noch vorhandener 3'-OH-Gruppen
5. Abspaltung von $R^8$
u.s.w.

Schema 6

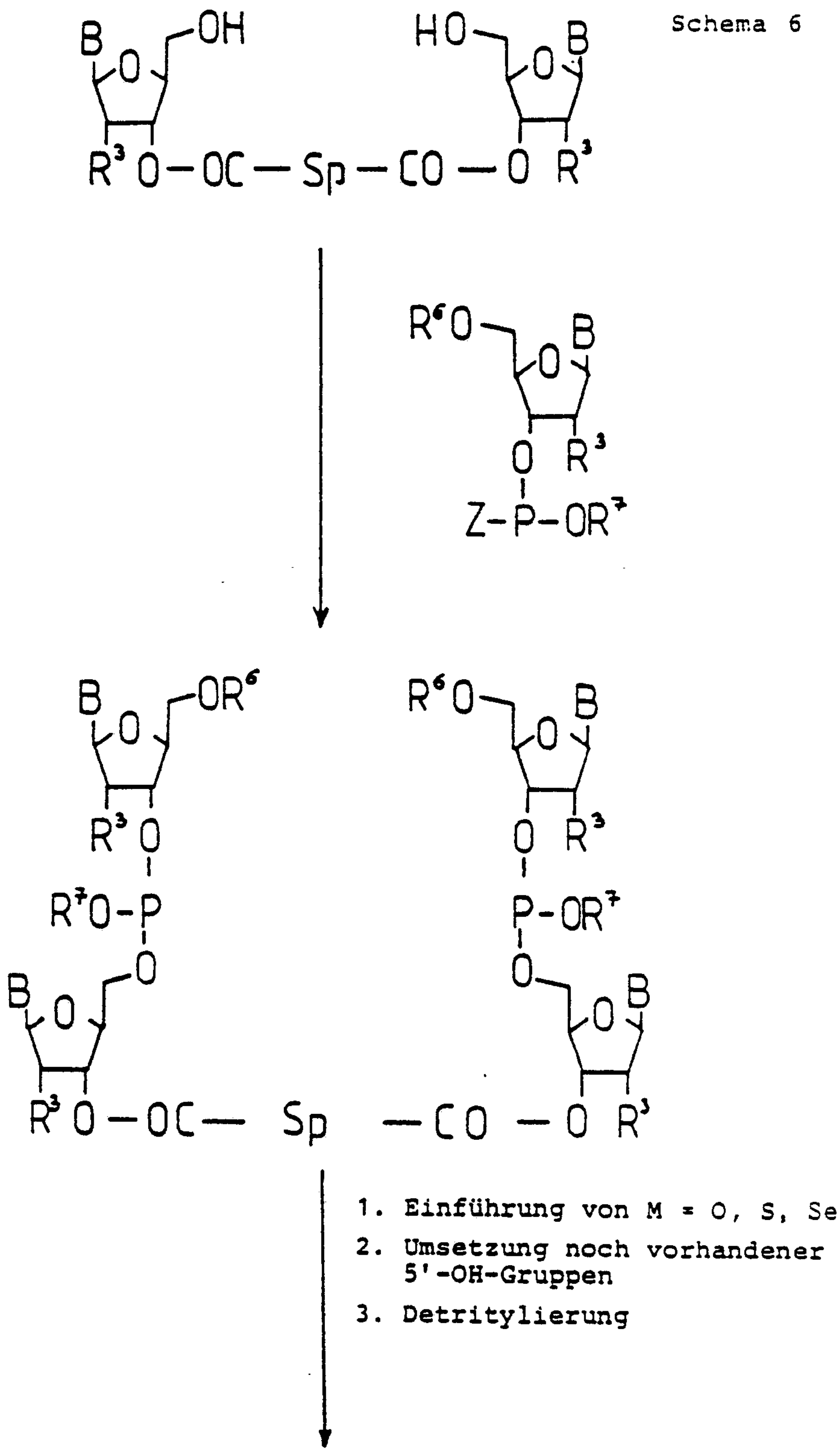
1. Einführung von M = O, S, Se
2. Umsetzung noch vorhandener 5'-OH-Gruppen
3. Detritylierung

Schema 6
(Fortsetzung

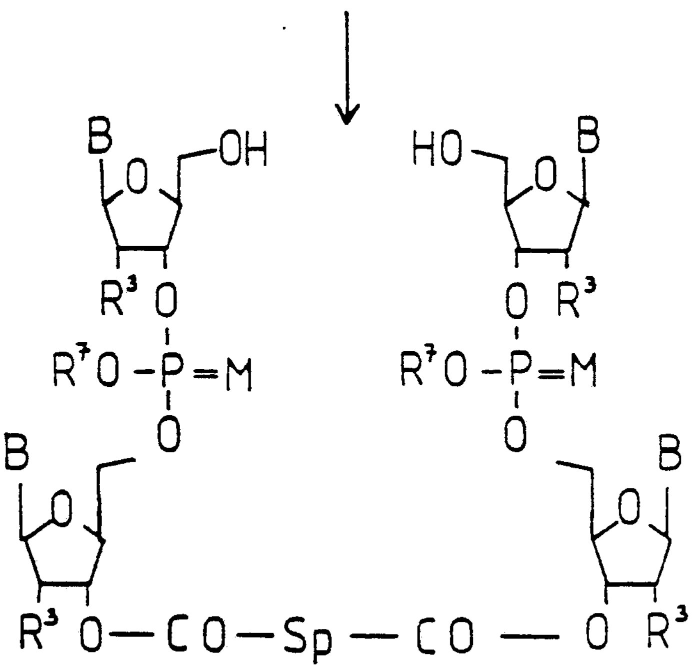

Schema 7

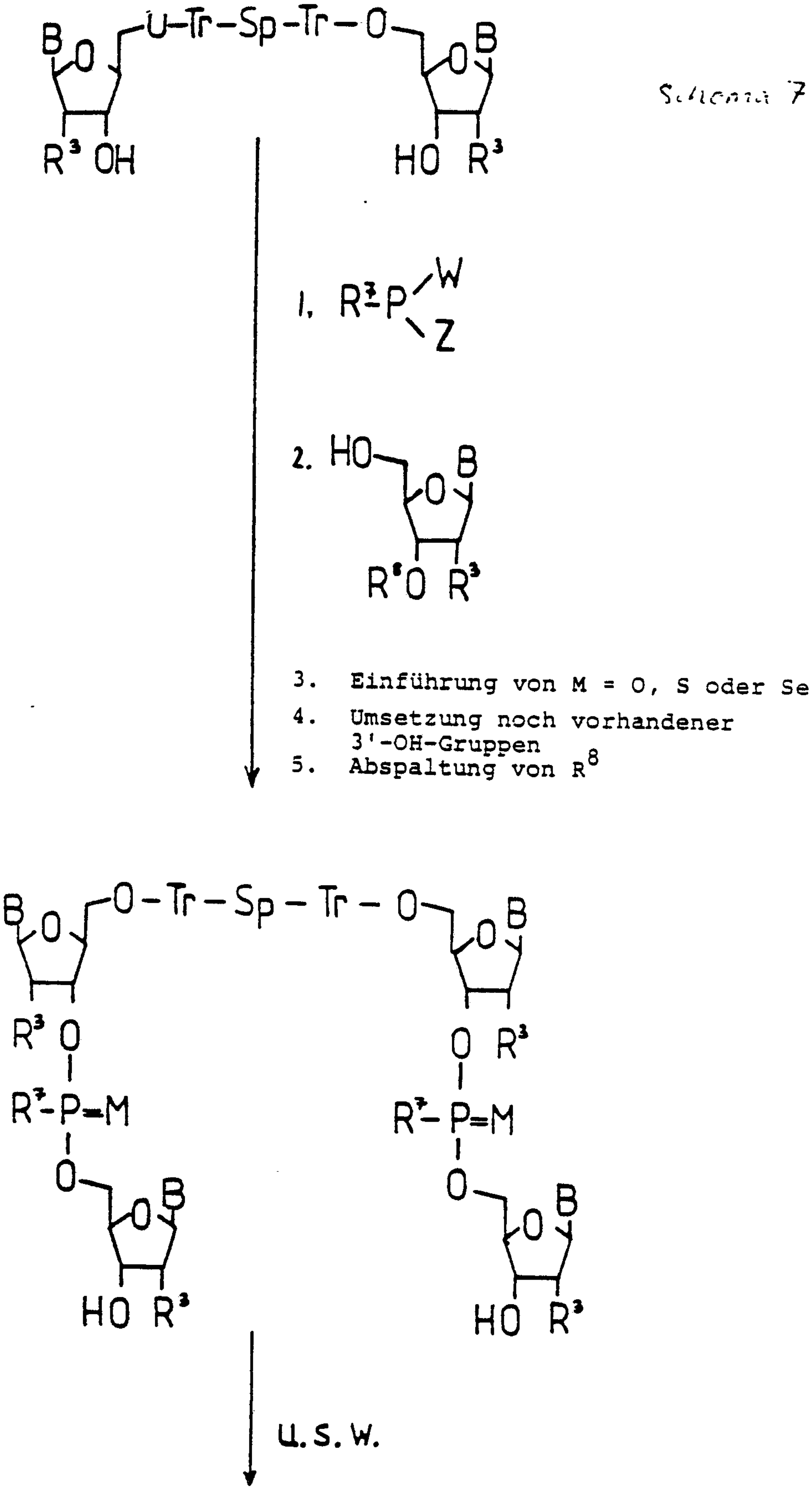
1.
2.
3. Einführung von M = O, S oder Se
4. Umsetzung noch vorhandener 3'-OH-Gruppen
5. Abspaltung von $R^8$
u.s.w.

Schema 4

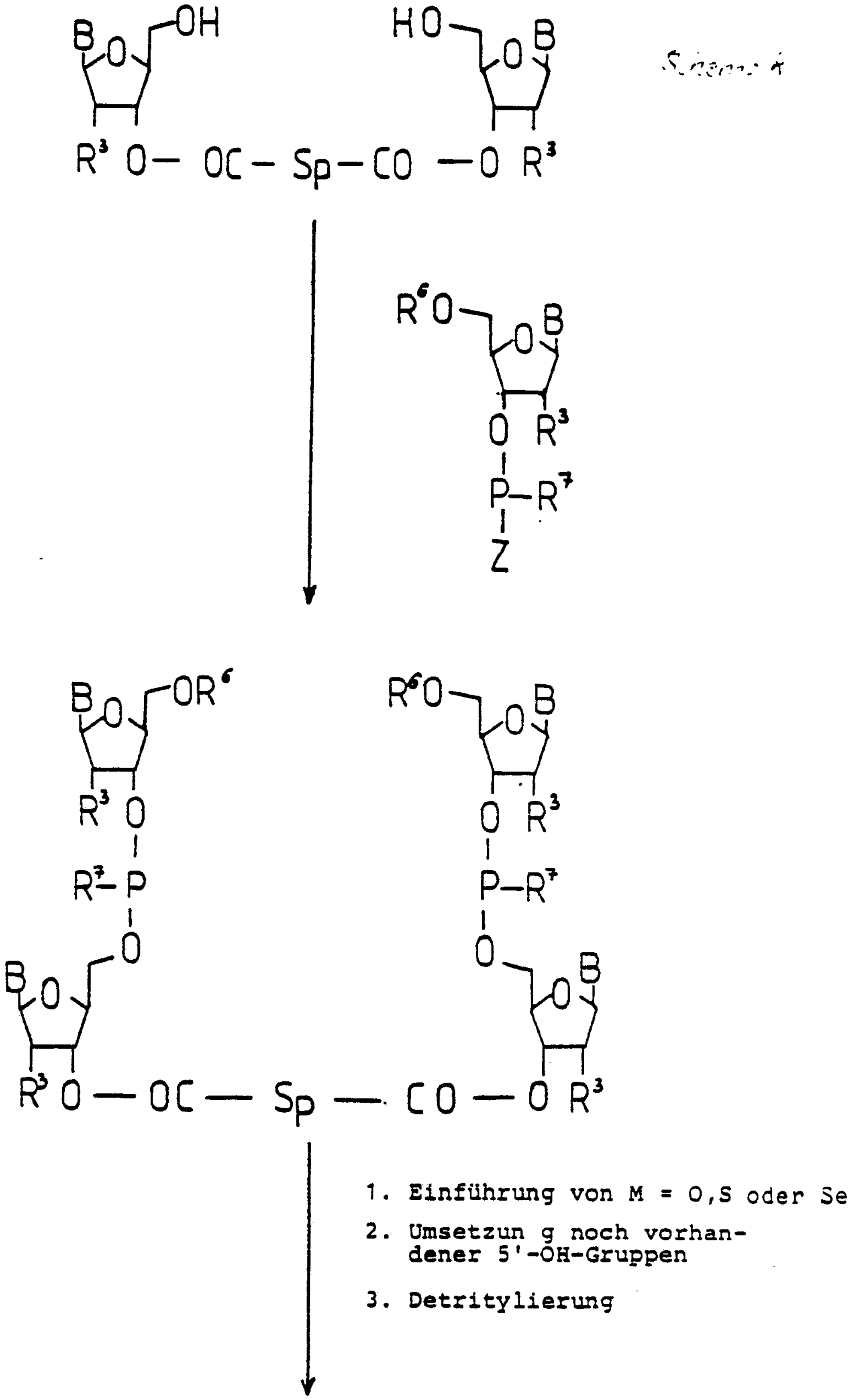
1. Einführung von M = O,S oder Se
2. Umsetzun g noch vorhandener 5'-OH-Gruppen
3. Detritylierung

Schema 8
(Fortsetzung)

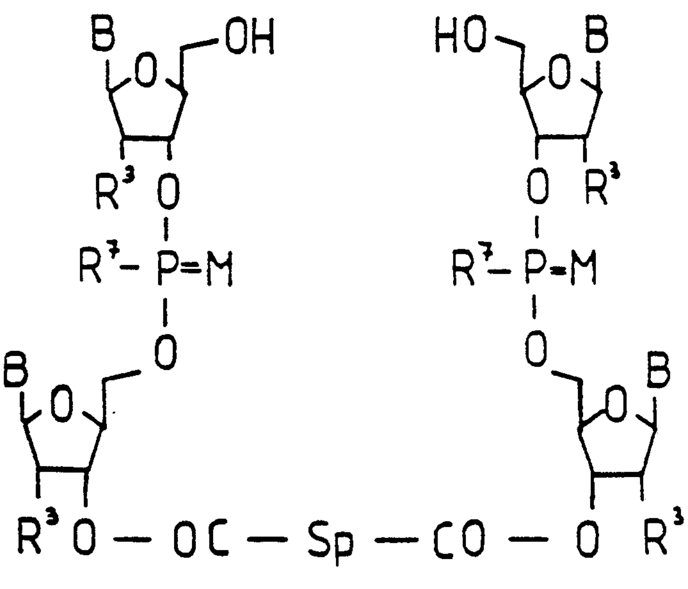

Schema 9

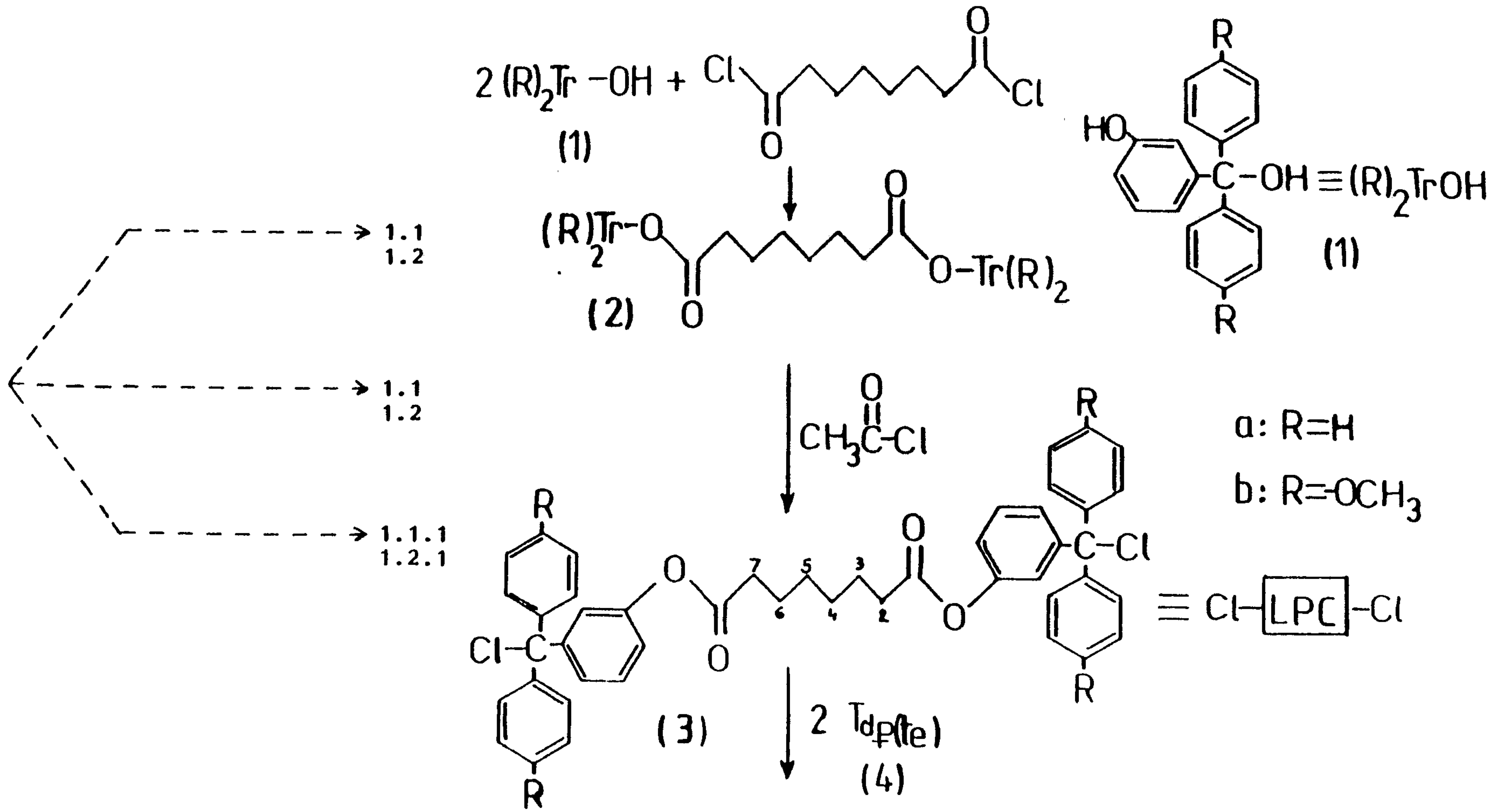


(1) (2) (3) (4)

Schema 9
(Fortsetzung I)

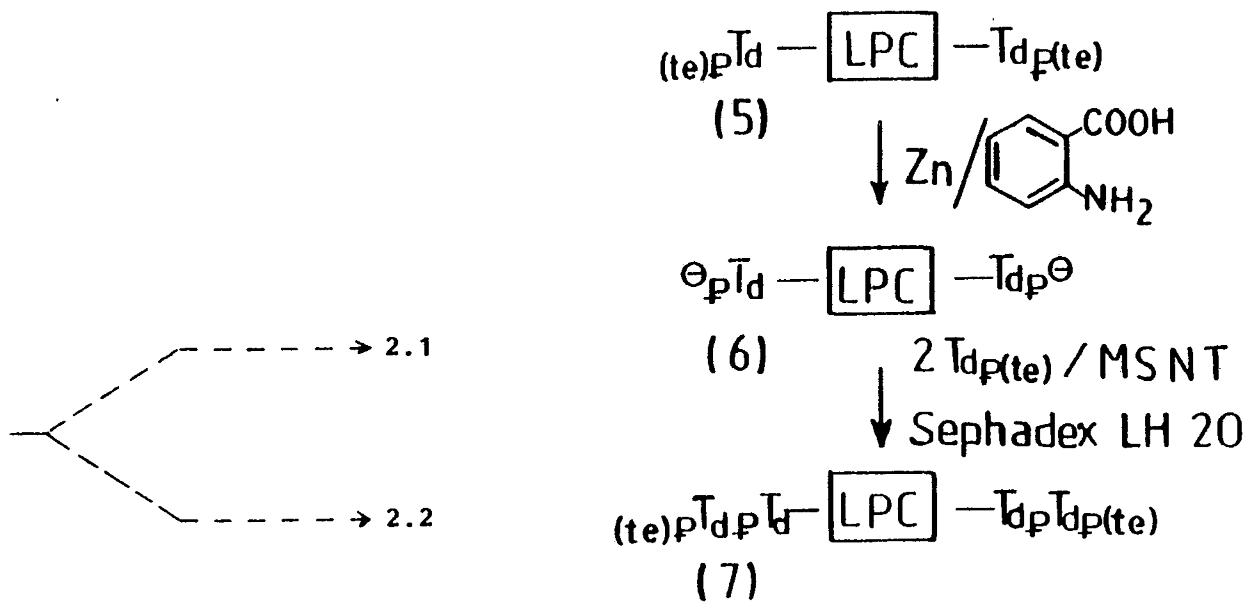


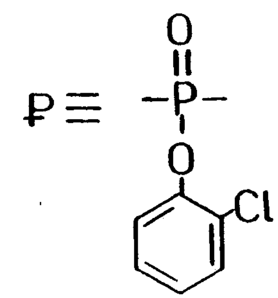

te = $-OCH_2CCl_3$

Schema 9
(Fortsetzung)